(19) Europäisches Patentamt
European Patent Office
Office européen des brevets

(11) Publication number: 0 144 744
A2

# (12) EUROPEAN PATENT APPLICATION

(21) Application number: 84113237.6

(22) Date of filing: 03.11.84

(51) Int. Cl.[4]: G 01 N 33/53
G 01 N 33/537, G 01 N 33/542

(30) Priority: 30.11.83 US 556633
30.11.83 US 556644

(43) Date of publication of application:
19.06.85 Bulletin 85/25

(84) Designated Contracting States:
AT BE CH DE FR GB IT LI LU NL SE

(71) Applicant: MILES LABORATORIES, INC.
1127 Myrtle Street
Elkhart Indiana 46514(US)

(72) Inventor: Blake, Diane A.
504 Ridge Wood Lane
Franklin, TN 37064(US)

(72) Inventor: Boguslaski, Robert C.
52118 Iris Court
Elkhart, IN 46514(US)

(72) Inventor: Carrico, Robert J.
54256 Silver St.
Elkhart, IN 46514(US)

(72) Inventor: Skarstedt, Mark T.
53823 County Road 3 North
Elkhart, IN 46514(US)

(74) Representative: Adrian, Albert, Dr. et al,
c/o BAYER AG Konzernverwaltung RP Patentabteilung
D-5090 Leverkusen 1, Bayerwerk(DE)

## (54) Specific binding assay based on enzyme cascade amplification.

(57) The present invention provides specific binding assay methods and reagent systems for the quantitative or qualitative determination of an analyte in a liquid medium based on heterogeneous or homogeneous protocols. In particular, the invention relates to the use of specific binding assay techniques wherein the label is a participant in or a modulator of an enzymatic reaction whereby a first enzyme acts on a first substrate to produce a second enzyme. The second enzyme can be used to catalyze a detectable response or can act on a second substrate to produce a third enzyme. The label is chosen from the first substrate or a cofactor or modulator of the first enzyme. In one embodiment, the first substrate is a zymogen which when acted upon by the first enzyme produces a second enzyme. When the final enzyme product is used to catalyze the production of a detectable response proportional of the concentration of the analyte, amplification results providing a very sensitive assay.

EP 0 144 744 A2

# SPECIFIC BINDING ASSAY BASED ON ENZYME CASCADE AMPLIFICATION

## BACKGROUND OF THE INVENTION

### *1. FIELD OF THE INVENTION*

The development of specific binding assay techniques has provided extremely useful analytical methods for determining various organic substances of diagnostic, medical, environmental and industrial importance which appear in liquid mediums at very low concentrations. Specific binding assays are based on the specific interaction between the substance under determination, herein referred to as the "analyte", and a binding counterpart thereof. Where one of the analyte and its binding counterpart is an antigen, the assay is known as an immunoassay.

In conventional specific binding assay techniques, a sample of the liquid medium to be assayed is combined with a reagent system of various compositions. Such composition include a detectant reagent comprising a binding component incorporated with a label. The binding component of the detecting reagent interacts with other constituents of the reagent system and the analyte in the medium under assay to form a binding reaction system producing two species or forms of the detectant reagent, a bound-species and an unbound-species.

In the bound-species, the binding component, e.g., a hapten or antigen, in the detectant reagent is bound by a corresponding binding counterpart, e.g., an antibody, whereas in the unbound-species, the binding component is not so bound. The relative amount or proportion of the detectant reagent that results in the bound-species compared to the unbound-species is a function of the presence (or amount) of the analyte in the test sample.

Where the detectant reagent in the bound-species is essentially indistinguishable in the presence of the detectant reagent in the unbound-species by the system used to monitor the label, the bound-species and the unbound-species must be physically separated in order to complete the assay. This type of assay is referred to in the art as "heterogeneous". Where the bound-species and unbound-species forms of the detectant reagent can be distinguished in the presence of each other, a "homogeneous" format can be followed and the separation step avoided.

This invention relates to specific binding assay methods and reagent systems for the quantitative or qualitative determination of an analyte in a liquid medium based on heterogeneous or homogeneous protocols. In particular, the present invention relates to the use of specific binding assay techniques wherein the label is a participant in or a modulator of an enzymatic reaction.

## 2. *DESCRIPTION OF THE PRIOR ART*

The first highly sensitive specific binding assay to be discovered was the radioimmunoassay which employs a radioactive isotope as the label. Such an assay necessarily must follow the heterogeneous format since the monitored response of the label is not significantly changed in the unbound- and bound-species. Because of the inconvenience and difficulty of handling radioactive materials and the necessity of a separation step, homogeneous assay systems have been devised using materials other than radioisotopes as the label component, including enzymes, bacteriophages, metals and organometallic complexes, enzyme substrates, coenzymes, enzyme inhibitors, cycling reactants, spin radicals, organic and inorganic catalysts, prosthetic groups, chemiluminescent reactants, and fluorescent molecules.

British Patent No. 2,059,421A describes a method for determining a ligand or receptor which uses as the labeled component, a conjugate between (i) a ligand or a receptor and (ii) a primary enzyme that is itself capable of producing or removing a modulator for a secondary system or that is the first enzyme in an enzyme system that is capable of producing or removing a modulator for a secondary system. To complete the assay, the labeled component is determined by adding the primary enzyme and any other enzymes in the enzyme system, allowing the secondary system to function in the presence or absence (as appropriate) of the modulator, and determining a product of the secondary system. A suggestion is made to use the enzyme C3-convertase as the label. C3-convertase acts on the complement factor C3 to produce C3b. C3b, in the

presence of other components, will also convert C3 to C3b and is thus autoactivating. There are two disadvantages of this system that would prevent its use in a practical laboratory immunoassay; first the label is an unstable enzyme and second no C3b substrates are available to provide a chromogenic detection system.

Generally representative of homogeneous specific binding assays are those described in the following references: U.S. Patent Nos. 4,134,792; 4,226,978; 4,230,797; 4,238,195; 4,238,565; 4,208,479; 4,233,401; 4,265,834; 3,817,837, 4,043,872; 4,233,402; 4,160,645; and 3,690,834.

## SUMMARY OF THE INVENTION

In accordance with the present invention, a specific binding assay method and reagent system are provided wherein the label component employed in the detectant reagent is a participant in or a modulator of an enzyme cascade reaction wherein a first enzyme acts on a first substrate to produce a second enzyme. The production of the second enzyme can be followed or the second enzyme can act on a second substrate to produce a third enzyme. Whose production may be followed.

The assay may be of the heterogeneous or homogeneous type, the latter being preferred. Following a homogeneous format, the progress of the assay is monitored by measuring the modulating effect of the specific binding of the detectant reagent with a binding counterpart thereof on the production of the second, third or subsequent enzyme. Such binding in the usual case causes an inhibition of enzyme production, however, enhancement of enzyme production is also contemplated. The method is applicable to the

determination of a wide variety of analytes and to use in a wide variety of modes, from liquid test systems to solid-state test devices, and from manual to automated systems.

In the homogeneous format, the method for determining an analyte in a liquid medium preferably comprises forming a reaction mixture by combining the liquid medium with a reagent system which system includes a binding counterpart for the analyte and a detectant reagent. The detectant reagent comprises the analyte, or a binding analog of the analyte, incorporated with the label. The combination of the liquid medium with the reagent system results in the formation of a bound-form of the detectant reagent, wherein the detectant reagent is bound to the binding counterpart, and an unbound-form of the detectant reagent wherein the detectant reagent is not so bound; the ability of the label to participate in or modulate the enzyme cascade reaction being measurably different when the detectant reagent is in the bound as compared to the unbound-form. The activity of the second, third or subsequent enzyme (formed in the enzyme cascade reaction) in the reaction mixture is then measured as a function of the analyte in the liquid medium.

All enzymes can bring about amplification, i.e., one enzyme molecule can catalyze formation of thousands of product molecules from a given substrate in a given period of time. However, true amplification, increased sensitivity of the assay system, results when the enzyme product of the enzyme reaction system is used in a second reaction to catalyze the formation of second product. At best, prior art methods for monitoring competitive protein binding assays are based on the use of enzymatic reactions which do not

produce an enzyme product. Therefore true amplification, an increased sensitivity of the assay system to the concentration of the analyte of interest, is not possible. The advantage of the present invention is that since the reaction product is also an enzyme, it can, in turn, catalyze the formation of thousands of molecules of a second product. If this product is detectable, the concentration can be related to that of the analyte resulting in one step amplification. When this second product is a third enzyme which can catalyze the formation of a third product which can give a detectable response proportional to the concentration of the analyte, "double" amplification results. This double amplification cascade will allow an extremely sensitive assay. The enzyme cascade can be continued to allow further amplification if the third product is also an enzyme.

## BRIEF DESCRIPTION OF THE DRAWINGS

Fig. 1 is a flow chart showing the zymogen activation immunoassay system demonstrated in Example 1, where the zymogen Factor X and Factor X activating enzyme from Russell's Viper Venom constitute the label/first enzyme pair. Addition of an antibody for the analyte inhibits participation of the zymogen label in the enzyme cascade. Detection is accomplished through the action of the enzyme product (second enzyme) on a chromogenic substrate to produce color. In Fig. 1, Factor X activating enzyme from Russell's Viper Venom is abbreviated RVV; antibody is abbreviated Ab; and analyte, A. A-Factor X denotes the detectant reagent; Ab:A and Ab:A-Factor X denote antibody-bound forms.

Fig. 2 is a graph showing the rate of activation of the zymogen-substrate to the enzyme product with

the addition of antibody to the analyte. In Example 1 the substrate/first enzyme pair are Factor X/activating enzyme from Russell's Viper Venom, the second enzyme is Factor Xa; and the analyte is theophylline. The rate of activation is measured as the slope of a plot of absorbance versus $(time)^2$. On the graph the rate of activation in absorbance per $(minutes)^2$ is plotted vs. concentration of antibody to theophylline added in microliters per milliliter (ml) of assay solutions. A microliter of purified antibody preparation contains 5.62 μg of protein.

Fig. 3 is a dose-response curve showing the rate of activation of substrate to enzyme product with the addition of the free analyte, as described in Example I. This assay is performed in the presence of antibody to theophylline. On the graph the rate of activation in absorbance per $(minutes)^2$ is plotted versus concentration of analyte added in micromoles.

Figure 4 is a flow chart showing the blood coagulation zymogen immunoassay system where the zymogen Factor X and Factor X activating enzyme from Russell's Viper Venom constitute the label/first enzyme pair. Addition of an antibody for the analyte inhibits participation of the zymogen label in the enzyme cascade. Factor Xa, the second enzyme, acts on prothrombin to produce thrombin, the third enzyme. Detection is accomplished through the action of the prothombin on a chromogenic substrate to produce color. In the flow chart, Factor X activating enzyme from Russell's Viper Venom is abbreviated RVV; analyte is abbreviated A, and antibody to the analyte, Ab. A-Factor X denotes the detectant reagent; Ab:A and Ab:A-Factor X are designations used for the bound forms of the analyte and detectant reagent respectively. In the example, the analyte is biotin and a natural binding protein, avidin is used in place of an antibody for the analyte.

Figure 5 is a graph showing the percentage of activity of the first zymogen substrate, Factor X, remining after the addition of avidin, a binding protein for the analyte biotin. The rate of activation is measured as the slope of a plot of absorbance at 406 nanometers versus $(\text{time in minutes})^3$.

Figure 6 is dose response curve showing the rate of Factor X activation to Factor Xa, measured as described in Example 3, with the addition of the free analyte, biotin, in nanomoles/liter (nM). The assay is performed in the presence of avidin, a protein which binds to biotin. The rate is in units of absorbance per $(\text{minutes})^3$.

## DESCRIPTION OF THE PREFERRED EMBODIMENTS

In the context of this disclosure, the following terms shall be defined as follows unless otherwise indicated:

Analyte - the substance, or class of related substances, whose presence or amount in a liquid medium is under determination.

Binding counterpart of the analyte - any substance, or class of substances, which has a specific binding affinity, normally reversible, for the analyte.

Specific binding analog of the analyte - any substance, or class of substances, which behave similarly to the analyte with respect to binding by a binding counterpart of the analyte.

Reagent system - a composition, test device, test kit, or other physical arrangement or combination of reagents for use in performing the present assay method.

Label - participant in or modulator of an enzymatic reaction wherein one enzyme acts on a first substrate to produce a second enzyme. The label is chosen from the first substrate or a cofactor or modulator of the first enzyme. The label is a particularly novel aspect of the invention and is defined in more detail in the following description.

Detectant reagent - comprises a binding component incorporated with the label.

The general enzyme cascade reaction contemplated is one which follows the equation:

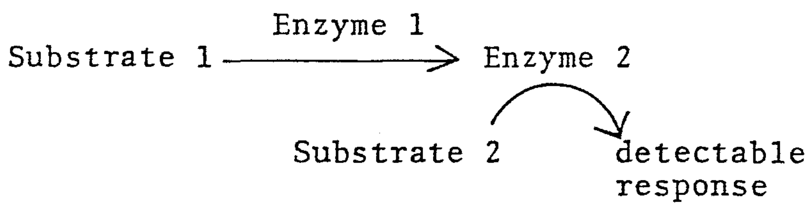


The first enzyme (Enzyme 1) may catalyze addition, cleavage or rearrangement of the substrate to produce the second enzyme. The enzyme formed (Enzyme 2) can be used as a catalyst for a further reaction resulting in a detectable response with subsequent true amplification.

The double enzyme cascade system contemplated is one which follows the equation:

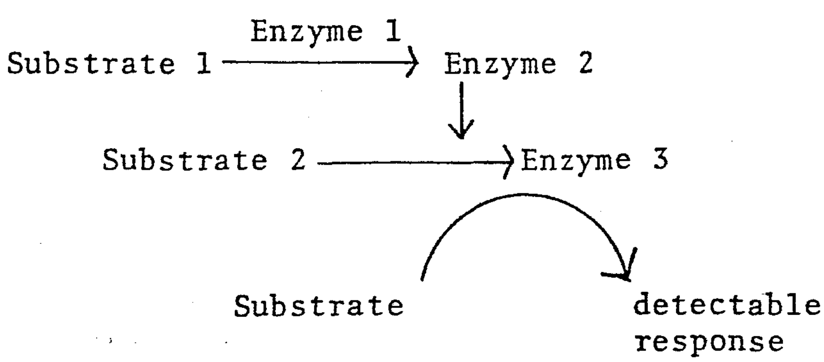

As before, the first enzyme (Enzyme 1) can catalyze addition, cleavage or rearrangement of a first substrate to produce a second enzyme (Enzyme 2). The second enzyme then catalyzes the addition, cleavage or rearrangement of a second substrate to produce a third enzyme. The enzyme formed (Enzyme 3) can be used as a catalyst for a further reaction resulting in a detectable response. This double-enzyme cascade system results in two steps or double amplification. Multi-step amplification could be obtained if the third product is also an enzyme.

There are a number of naturally occurring multiple cascade reactions which are useful in the present invention. Examples of such multiple cascade reactions are found in the blood coagulation cascade, the hemolymph coagulation system of the horseshoe crab, zymogens of digestive proteases and a phosphorylation cascade. The first enzyme/first substrate pair must be chosen such that the enzyme product (Enzyme 2) can catalyze the formation of a third enzyme by its action on a second substrate. Preferably reaction components are chosen so that there are specific substrates which are acted upon by Enzyme 3 to provide a detectable response.

*1. Label*

Once a desirable first enzyme/substrate pair is chosen, the present invention is based on the use of a label component in the detectant reagent selected from the first substrate, a required cofactor or any modulator of the first enzyme reaction.

*a. Substrate as the Label*

The first substrate will usually be a proenzyme or a regulatory enzyme which may be activated or inactivated. Commonly, cleavage of addition of

phosphate, an adenyl group, or a polypeptide will cause activation of a proenzyme or regulatory enzyme, or inactivation of a regulatory enzyme.

When the cleavage of a polypeptide group from a proenzyme is catalyzed by a first enzyme to produce a second enzyme, the proenzyme-substrate is called a zymogen. Zymogens are particularly preferred labels.

Suitable zymogen/first enzyme pairs include chymotrypsinogen/trypsin, trypsinogen/enteropeptidase, procarboxypeptidase A/trypsin, procarboxypeptidase B/trypsin, proelastase/trypsin, prothrombin/Factor Xa, plasminogen/urokinase, and Factor X/Factor X activating enzyme from Russell's Viper Venom.

Another group of suitable substrates is that of regulatory enzymes which may be activated or inactivated by the addition or cleavage of phosphate or adenyl groups (See e.g., Table 1).

TABLE 1

| ADDITION | ACTION | SUBSTRATE | ENZYME 1 | ENZYME 2 |
|---|---|---|---|---|
| $-PO_4$ | inactivates | glycogen synthetase (I) | protein kinase | glycogen synthetase (D) |
| $-PO_4$ | inactivates | phosphorylase a | phospho-protein phosphatase | phosphorylase b |
| $-PO_4$ | activates | phosphorylase b | phosphory-lase b kinase | phosphorylase a |
| adenyl | inactivates | glutamine synthetase | glutamine synthetase adenylyl-transferase | glutamine synthetase AMP |
| CLEAVAGE | | | | |
| $-PO_4$ | activates | glycogen synthetase (D) | phospho-protein phosphatase | glycogen synthetase (I) |

When double amplification is desired, suitable zymogen/first enzyme pairs include: 1) from the blood coagulation cascade: Factor X/Factor X activating enzyme from Russell's Viper Venom, Factor X/Factor IXa, Factor IX/Factor XIa, Factor XI/Factor XII; 2) from the horseshoe crab hemolymph system: Factor B/Factor X activating enzyme from Russell's Viper Venom; 3) from the digestive zymogens: trypsinogen/-enterokinase and 4) from the phosphorylation cascade: phosphorylase kinase/protein kinase. In each case, the first enzyme acts upon the first substrate to produce a second enzyme which second enzyme acts on a second substrate to produce a third enzyme, resulting in a double enzyme cascade system. For example: Factor X activating enzyme from Russell's Viper Venom (the Factor X activating enzyme from Russell's Viper Venom

is referred to herein as RVV) acts on Factor X to produce Factor Xa, an enzyme which in turn can act on prothrombin to produce thrombin; in a second enzyme, enterokinase acts upon trypsinogen to produce trypsin, an enzyme which in turn acts on proelastase to produce elastase. In each case, the third enzyme, thrombin or elastase, can catalyze the production of a third product which provides a detectable response which can be related to the concentration of the analyte of interest.

In a preferred embodiment, RVV acts on Factor X to produce Factor Xa; Factor Xa then acts on prothrombin to produce a third enzyme, thrombin. A suitable chromogenic substrate for thrombin is provided so that a detectable response is generated which can be related to the concentration of the analyte.

For double amplification, another group of suitable substrates is that of regulatory enzymes which may be activated or inactivated by the addition or cleavage of a phosphate. One suitable first substrate/first enzyme pair is phosphorylase b kinase/-protein kinase. Protein kinase catalyzes the addition of phosphate to phosphorylase b kinase to produce phosphorylase a kinase, the active form of the enzyme; phosphorylase a kinase acts on a second substrate, phosphorylase b, to produce a third enzyme, phosphorylase a. A suitable substrate for phosphorylase a is provided, completing the double amplification cascade, so that a detectable response is generated which can be related to the concentration of the analyte.

*b. Cofactor as the Label*

If the first substrate/first enzyme pair chosen requires a cofactor to produce the second enzyme, that cofactor can be used as the label. Suitable cofactors are adenosine monophosphate (AMP), adenosine triphosphate (ATP), nicotine adenine dinucleotide (NAD) and other nucleotide coenzymes. For example ATP is required for the phosphorylation of glycogen synthetase (I) by protein kinase and of phosphorylase b by phosphorylase b kinase. 3',5'-cyclic AMP modulates the phosphorylation of glycogen synthetase (I) to form glycogen synthetase (D). In the double amplification casades, 3',5'-cyclic AMP modulates the addition of a phosphate group (phosphorylation) to phosphorylase b kinase by protein kinase. In any of these reactions the cofactor can be used as a label. In the blood coagulation cascade, Factor VIIIa, $Ca^{+2}$ and phospholipid are required as cofactors for the activation of Factor X by Factor IXa.

*c. Modulator of the First Enzyme as the Label*

Any substance which modulates the catalytic action of the first enzyme on the first substrate to produce a second enzyme may also be used as the label. Commonly, the modulation takes the form of inhibition, however, enhancement of the production of the second enzyme is also contemplated.

These modulators include natural inhibitors, synthetic inhibitors, and antibodies or fragments thereof for the first enzyme. One natural inhibitor of a single amplification enzyme cascade is soybean trypsin inhibitor. Soybean trypsin inhibits the action of trypsin on chymotrypsinogen, thereby decreasing the production of chymotrypsin. One natural inhibitor of

a double amplification enzyme cascade is $\alpha_2$-macroglobulin. $\alpha_2$-Macroglobulin inhibits the action of RVV on Factor X, thereby decreasing the production of Factor Xa.

For examples, such compounds as

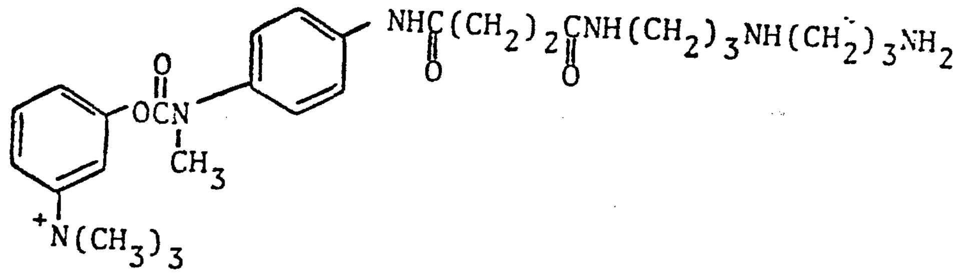

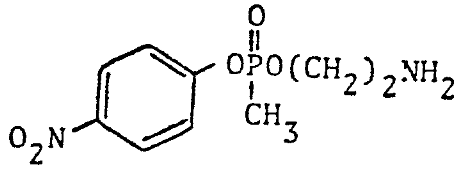

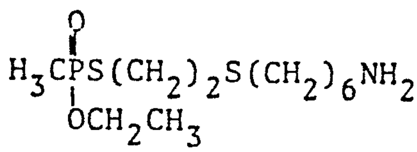

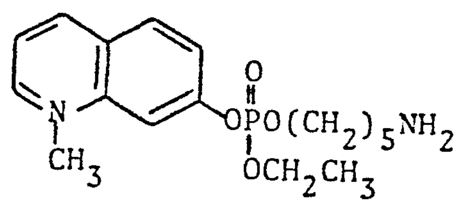

will inhibit the ability of trypsin to catalyze the conversion of chymotrypsinogen to chymotrypsin. Such compounds as diisopropylfluorophosphate, o-phenanthreoline, ethylenediaminetetraacetate, and ethyleneglycol bis(β-aminoethylether)-N,N,N',N'-tetraacetic acid will inhibit the ability of RVV to catalyze the conversion of Factor X to Factor Xa. This first enzyme reaction can be used in a multienzyme cascade when a suitable second substrate for Factor Xa is provided.

As a modulator label, one can also use an antibody raised to the first enzyme, or a fragment of such antibody. Techniques used to obtain the antibody are well known to those skilled in the art. In the reaction mixture, such an antibody label will bind the first enzyme, affecting the normal interaction of the first enzyme and first substrate, thereby decreasing or increasing the production of the second, third or subsequent enzyme. Natural binding proteins can be used in a similar manner. For example, avidin is a natural protein which binds biotin.

*2. Detectant Reagent*

The detectant reagent comprises two principal components which are coupled to one another either by direct chemical linkage, e.g., by covalent bonds or noncovalent binding, or by indirect attachment, e.g., through microcapsules or liposomes. One component is the binding component, whose function is well-known in the art. The binding component and the analyte in the sample participate in the specific binding reaction system. Depending on the particular assay format followed, the binding component will usually be the analyte itself, a specific binding analog of the analyte, or a binding partner for the analyte. The choice of a particular binding component in a given assay and methods for incorporating same into the detectant reagent are matters of ordinary skill in the art.

The other component of the detectant reagent is the novel label component of the present invention, a participant in or modulator of the first enzyme reaction in an enzyme cascade reaction. In choosing the sites of attachment on the binding component and the label of the present invention, the important considerations on a general level are (1) preservation of the ability of the linked binding component to participate effectively

in the selected binding assay system, and (2) preservation of the ability of the linked label to participate in or modulate the first enzymatic reaction; in both cases, to the extent that a useful assay will result for the particular analyte under assay and for the particular concentrations in which such is to be detected. Commonly, the linking group will comprise a chemical bond, usually a single bond, or a chain containing between 1 to 20, more commonly 1 to 10 carbon atoms and 0 to 10, more commonly 1 to 5, heteroatoms selected from nitrogen, oxygen, and sulfur. Further details regarding the selection of linking groups may be found in U.S. Patents Nos. 4,238,565 and 3,817,837.

In the most usual case, the participant in or modulator of the first enzyme reaction in an enzyme cascade used as the label in the present invention and the binding component to be coupled therewith will have available amino and carboxyl functionalities for coupling by conventional peptide condensation reactions. Where the chosen label is a protein, e.g., when a substrate zymogen, or a modulator such as an antibody or other natural proteinaceous inhibitor or stimulator is used as the label, it will have numerous active amino and carboxyl groups to participate in peptide condensation. Oftentimes, the analyte will itself contain an amino or carboxyl group useful for coupling to the label by peptide condensation, such as where the analyte is a protein or polypeptide or is a hapten that is a primary amine or carboxylic acid. Where the analyte does not have an available functionality for coupling to the label, such can be readily introduced by forming an amino or carboxyl derivative of the analyte. Typical analyte derivatives of this type (i.e., specific binding analogs of the analyte) and further details concerning the formation of the detectant

reagent by peptide condensation and equivalent techniques are provided in U.S. Patent No. 4,226,992, particularly in columns 3-10 thereof, which is incorporated herein by reference.

Conventional peptide condensation reactions include the carbodiimide reaction [*Science 144*:1344 (1964)], the mixed anhydride reaction [Erlanger et al, *Methods in Immunology and Immunochemistry*,. ed. Williams and Chase, Academic Press (New York 1967) p. 149], and the acid azide and active ester reactions [Kopple, *Peptides and Amino Acids*, W. A. Benjamin, Inc. (New York 1966)]. See also for a general review *Clin. Chem. 22*:726 (1976). In a preferred embodiment where Factor X is the label, the analyte is attached to this zymogen-first substrate by covalent conjugation to the carbohydrate side chain attached to asparagine 36 of the bovine Factor X heavy chain, via periodate oxidation and subsequent reductive amination with an amino group on the analyte.

When a cofactor or synthetic inhibitor is used as the label, the analyte or its binding analog can be linked through a free amino group to form the detectant reagent. It will be recognized that other well known methods are available for coupling the analyte and label to form the detectant reagent of the present invention. For example, bifunctional reagents can be used to couple amines to amines, e.g., bisisocyanates, bisimidoesters, and glutaraldehyde [*Immunochem. 6*:53(1969)]. Functional groups on the analyte, or binding analog thereof, and on the label other than amino and carboxyl groups can be used as the site of attachment depending on the synthetic approach selected. These synthetic routes are available to one of ordinary skill in the art from the literature.

3. *Assay Techniques*

In broad principle, the present assay method may be performed according to any of the conventional homogeneous or heterogeneous formats. Homogeneous formats are generally preferred since they are particularly simple to perform. However, in those circumstances where the effect of the detectant reagent on the enzyme cascade reaction is essentially indistinguishable between the bound-species and the unbound-species, a heterogeneous format will be followed in order to perform an assay.

*a. Homogeneous Formats*

In the homogeneous assay technique, i.e., an assay technique which does not require a physical separation of the bound-species and the unbound-species, reaction between the binding component in the detectant reagent and a corresponding binding counterpart causes a measurable change, either in a positive or a negative sense, in the label's participation in or modulation of the first enzyme reaction in an enzyme cascade. The distribution of the detectant reagent between the bound-species and the unbound-species is usually differentiated by the inability or altered ability of the label to affect enzyme production when in the bound-species. Several manipulative techniques are available for carrying out a homogeneous assay with a common technique being the competitive binding technique. In the competitive binding technique, the sample is combined with a binding counterpart of the analyte, a detectant reagent comprising the label coupled to the analyte or a specific binding analog thereof, and the other components necessary to produce the second enzyme and

thereafter measuring production of the second or third or subsequent enzyme in the reaction mixture. Alternatively, following a sequential test format, the sample and the analyte binding counterpart are first combined and thereafter the detectant reagent added.

Homogeneous formats are applicable to the determination of any desirable analyte, including antigenic proteins and polypeptides and haptens. Antibodies may be determined as antigenic proteins by using either an anti-antibody antibody as the binding counterpart, (this antibody assay will only be class specific since it is unable to distinguish antibodies according to their antigen specificities), or an antigen or hapten as the binding counterpart. In the latter case, where antibodies, or other binding proteins, receptors, or binding materials in general, are determined according to their counterpart specificities, a direct technique can be used. The liquid medium is combined with a detectant reagent comprising the label coupled to a binding counterpart of the analyte, and the other components necessary to produce the second, third or subsequent enzyme, and thereafter final enzyme produced is again measured in the reaction mixture. In this case, the "analyte" may be antibodies having specificity for a particular antigen or hapten (which serves, in its native form or a binding analog form, as the binding counterpart in the detectant reagent), or may be the binding capacity of the test saple to bind a particular substance due to the presence in the sample of a particular binding protein, receptor, carrier substance, or the like, e.g., triiodothyronine or thyroxine binding capacity ($T_3$ uptake or $T_4$ uptake).

In general, when following the homogeneous assay technique, the components of the assay reaction, i.e., the liquid medium suspected of containing the analyte, the detectant reagent, other components necessary to product second, third or subsequent enzyme, and, if necessary, a binding counterpart of the analyte, may be combined in any amount, manner, and sequence, provided that enzyme production is measurably altered when the liquid medium contains the analyte in an amount or concentration of significance to the purposes of the assay. Preferably, all of the components of the specific binding reaction are soluble in the liquid medium. Additionally, the reaction mixture will be formed to contain a conventional indicator composition which produces a detectable response, e.g., light absorption, color, fluorescence, chemiluminescence, and so forth, as a function of the production of the second enzyme.

*b. Heterogeneous Formats*

The present assay method can also be applied to the conventional heterogeneous type assay techniques wherein the bound- and unbound-species of the detectant reagent are separated and the label component in one or the other is determined. The reagent system for performing such a heterogeneous assay can take many different forms. In general, such a system comprises three basic constituents, which are (1) the analyte to be detected, (2) a binding counterpart of the analyte and (3) the detectant reagent, along with the other components necessary to produce the second, third or subsequent enzyme. The binding reaction constituents are combined simultaneously or in a series of additions and with an appropriate incubation period or periods, the detectant reagent becomes bound to its corresponding

binding counterpart such that the extent of binding, i.e., the ratio of the amount of detectant reagent bound to a binding counterpart (the bound-species) to that unbound is a function of the amount of analyte present. The bound- and unbound-species are physically separated and the amount of label present in one thereof is determined by measuring production of the second, third or subsequent enzyme therein and comparing such to a negative control or standard results, e.g., a standard curve.

Various means of performing the separation step and of forming the binding reaction systems are available in the art. Separation can involve such conventional techniques as those involving what is commonly known as a solid-phase antibody or antigen, a second antibody, or a solid-phase second antibody, as well as the use of immune complex precipitation agents, absorbents, and so forth. Binding reaction systems that can be followed include the so-called competitive binding technique, the sequential saturation technique, the "sandwich" technique, and so forth. Further details concerning the various known heterogeneous systems are readily available in the literature, e.g., U.S. Patent No. 4,230,797.

It is contemplated that manipulative schemes involving other orders of addition and other binding reaction formats can be devised for carrying out homogeneous and heterogeneous specific binding assays without departing from the inventive concept embodied herein.

4. *Analyte*

The present assay may be applied to the detection of any analyte for which there is a specific binding counterpart available. The analyte usually is a peptide, polypeptide, protein, carbohydrate, glycoprotein,

steroid, or other organic molecule for which a specific binding counterpart exists in biological systems or can be synthesized. The analyte, in functional terms, is ususally selected from the group comprising antigens and antibodies thereto; haptens and antibodies thereto; hormones, vitamins, metabolites and pharmacological agents, and their binding counterparts, and hybridizable nucleic acids, e.g., DNA and RNA. Usually, the analyte is an immunologically-active polypeptide or protein, usually having a molecular weight of between about 1,000 and about 10,000,000, such as an antibody or antigenic polypeptide or protein, or a hapten having a molecular weight of at least about 100, and usually less than about 1,500.

However, the assay is particularly useful where the size of the analyte is 10,000 daltons or less, more preferably less than 5,000; and most preferably less than 1,500. Of course, the enzymatic reaction chosen must be such that it does not interact with the analyte itself. For example, if the analyte is a protein and a non-specific proteolytic enzyme is used, the enzyme could act on the analyte causing deviations in the assay results.

Representative polypeptide analytes are angiotensin I and II, C-peptide, oxytocin, vasopressin, neurophysin, gastrin, secretin, bradykinnin, and glucagon.

Representative protein analytes include the classes of protamines, mucoproteins, glycoproteins, globulins, albumins, scleroproteins, phosphoproteins, histones, lipoproteins, chromoproteins, and nucleoproteins. Examples of specific proteins are prealbumin, $\alpha_1$-lipoprotein, human serum albumin, $\alpha_1$-acid glycoprotein, $\alpha_1$-antitrypsin, $\alpha_1$-glycoprotein, transcortin, thyroxine binding globulin, haptoglobin, hemoglobin, myoblogin, ceruloplasmin, $\alpha_2$-lipoprotein, $\alpha$-macroglobulin,

β-lipoprotein, erythropoietin, transferrin, hemopexin, fibrinogen the immunoglobulins such as IgG, IgM, IgA, IgD, and IgE, and their fragments, e.g., $F_c$ and $F_{ab}$, complement factors, prolactin, blood clotting factors such as filbrinogen, thrombin and so forth, insulin, melanotropin, somatotropin, thyrotropin, follicle stimulating hormine, leutinizing hormone, gonadotropin, thyroid stimulating hormone, placental lactogen, intrinsic factor, transcobalmin, serum enzymes such as alkaline phosphatase, lactic dehydrogenase, amylase, lipase, phosphatases, cholinesterase, glutamic oxalo-acetic transaminase, glutamic pyruvic transaminase, and uropepsin, endorphins, enkephalins, protamine, tissue antigens, bacterial antigens, and viral antigens such as hepatitis associated antigens (e.g., $HB_sAg$, $HB_cAg$ and $HB_eAg$).

Representative hapten analytes include the general classes of drugs, metabolites, hormones, vitamins, and the like organic compounds. Haptenic hormones include thyroxine and triiodothyronine. Vitamins include vitamins A, B, e.g., $B_{12}$, C, D, E and K, folic acid and thiamine. Drugs include antibiotics such as aminoglycosides, e.g., gentamicin,tobramycin, amikacin, sisomicin, kanamycin, and netilmicin, penicillin, tetracycline, terramycin, chloromycetin, and actino-mycetin; nucleosides and nucleotides such as adenosine diphosphate (ADP) adenosine triphosphate (ATP), flavin mononucleotide (FMN), nicotinamide adenine dinucleotide (NAD) and its phosphate derivative (NADP), thymidine, guanosine and adenosine; prostaglandins; steriods such as the estrogens, e.g., estroil and estradiol, steriods; and others such as phenobarbital, phenytoin, primidone, ethosuximide, carbamazepine, valproate, theophylline, caffeine, propranolol, procainamide, quinidine, ami-tryptiline, cortisol, desipramine, disopyramide,

doxepin, doxorubicin, nortryptiline, methotrexate, imipramine, lidocaine, procainamide, N-acetylprocainamide, amphetamines, catecholamines, and antihistamines.

5. *Reaction Mixture*

The liquid medium to be assayed can be a naturally occurring or artificially formed liquid suspected to contain the analyte, and usually is a biological fluid or a dilution thereof. Biological fluids that can be assayed include serum, plasma, urine, saliva, and amniotic and cerebrospinal fluids.

The binding reaction will in almost all cases be allowed to proceed under mild conditions. The reaction mixture will be in general an aqueous medium with any desirable organic cosolvents being present in minor amounts. The temperature of the reaction will be maintained at a constant level in normal circumstances throughout the incubation period and the enzyme measurement step. Temperatures will generally be between 5 and 50 degrees centrigrade (°C), more usually between 20 and 40 °C. Preferably the reaction will proceed at room temperature. The pH of the reaction mixture will vary between 5 and 10, more usually between 6 and 9. The concentration of various reagents will depend on the level of analyte expected in the test medium, with such level usually being between $10^{-3}$ and $10^{-12}$ Molar (M). As in the case of the previously described reaction parameters, selection is primarily based on empirically derived optimization balanced against the preferences and needs of the technician who will ultimately perform assays on a routine basis. None of the parameters therefore is of a critical nature to the present invention, rather they are all within the ordinary skill in the art.

*6. Reagent System*

The reagent system of the present invention comprises all of the essential chemical elements required to conduct a desired assay method encompassed by the present invention. The reagent system is presented in a commercially packaged form, as a composition or admixture where the compatability of the reagents will allow, in a test device configuration, or as a test kit, i.e., a packaged combination of one or more containers holding the necessary reagents. Included in the reagent system are the reagents appropriate for the binding reaction system desired, always requiring a detectant reagent and the other components necessary to produce the second, third or subsequent enzyme as defined hereinbefore. Such binding reaction reagents can include, in addition to the detectant reagent, a binding counterpart to the analyte, the enzymatic indicator composition, and so forth. Of course, the reagent system can include other reagents as are known in the art and which may be desirable from a commercial and user standpoint, such as buffers, diluents, standards, and so forth.

In particular, the present invention provides a test kit for determining an analyte in a liquid medium, comprising (a) a detectant reagent incorporating the label and the analyte or a binding analog thereof, (b) a binding counterpart, e.g., antibody of the analyte, (c) the other components of the enzymatic reaction necessary to produce the second, third or subsequent enzyme, and (d) optionally, an indicator composition which produces a detectable response as a function of the production of the final enzyme in the enzyme cascade.

The present invention will now be illustrated, but is not intended to be limited, by the following examples.

*Example 1*

A specific binding assay for the determination of theophylline as the analyte was devised using Factor X/Factor X activating enzyme from Russell's Viper Venom as substrate/first enzyme pair. Factor X is a zymogen which is cleaved by the activating enzyme present in Russell's Viper Venom (herein the activating enzyme is referred to as RVV) to form the enzyme, Factor Xa. Figure 1 is a flow chart showing the assay utilizing the enzymatic reaction.

$$\text{Factor X} \xrightarrow{\text{RVV}} \text{Factor Xa}$$

The substrate, Factor X, was chosen as the label. The detectant reagent was formed by preparing a conjugate of theophylline and Factor X through the available lysine groups on the zymogen. In the presence of antibody to theophylline, the reactivity of Factor X in the detectant reagent was inhibited; this inhibition could be reversed by the addition of free theophylline to the system. (See Figure 3.) The zymogen activation immunoassay system responded to changes in unbound analyte concentration to yield a dose-response curve for theophylline. Activation was measured by addition of a chromogenic substrate, S-2222 (a product of Kabi Vitrum AB and available in the U.S. from Helena Laboratories, Beaumont, TX), for the enzyme product, Factor Xa. The substrate undergoes a color change when acted upon by Factor Xa.

Absorbance was measured on a spectrophotometer (Hewlett Packard Model 8450) and plotted versus (time in minutes)$^2$ to yield a linear plot. Details of experimental protocols and results follow.

A. Preparation of Theophylline: Factor X Conjugates

Materials

The label, Factor X, was incorporated with the analyte, theophylline, as follows:

To 1 milligram (mg) of bovine Factor X (purchased from Sigma Chemical Co., St. Louis, MO) containing 0.42 micromoles (μmoles) lysine in 1 milliliter (ml) of 0.2 Molar (M) sodium carbonate, pH 8.5, was added 0.21 mg (0.84 μmoles) of a mixed anhydride of theophylline [prepared as described by Cook, C.E. *et al. Chem. Pathol. & Pharmacol, 13*:497-505 (1976)] in 0.4 ml *p*-dioxane. The mixture was stirred for four hours at 5 degrees Centigrade (°C) and the Factor X-theophylline conjugate was purified by dialysis against a solution containing 0.14 M sodium chloride and 0.05 M sodium citrate at pH 5.8 or by filtration through a Sephadex® G-25 (purchased from Pharmacia, Inc., Piscataway, NJ) column, equilibrated in the same buffer. The activity of column fractions was assayed as described below. The degree of theophylline substitution was calculated from optical density data taken at 280 nanometers (nm) and the Bradford protein assay [Bradford, M., *Anal. Biochem. 72*, 248 (1976)].

B. Characterization of Theophylline-Factor X Conjugates

Two Factor X derivatives were prepared as described above. The first conjugate, purified by dialysis, contained 15.5 moles of theophylline per mole of Factor X, corresponding to derivatization of

67% of the total lysines; the second conjugate, purified by gel chromatography, contained 9.5 moles of lysine per mole of protein (42% of lysines derivatized). Both conjugates showed a single precipitation line in radial immunodiffusion when tested against antitheophylline antibodies.

C. Purification of Anti-Theophylline Antibody

An anti-theophylline antibody was raised using standard polyclonal techniques and purified as follows:

Twenty-five mL of antitheophylline antiserum (Li, T. M. *et al. Clin. Chem. 27*, 22-26 1981) was brought to 50% saturation with ammonium sulfate, and the precipitated protein was collected by centrifugation. After suspension in 10 millimolar (mM) potassium phosphate at pH 7.0, the solution was again brought to 50% saturation with ammonium sulfate. The precipitate was collected and resuspended in 10 ml of 10 mM potassium phosphate buffer. After dialysis against 4 x 1 liters of 10 mM potassium phosphate buffer, pH 7.5, the dialysate was centrifuged to remove precipitated protein.

Protein A-agarose was prepared by swelling 1 gram of the dried gel (purchased from Sigma Chemical Co.) in 100 mM sodium phosphate pH 7.0 and washing the swollen resin in 200 mL of the same buffer. The gel preparation was packed in a 1.2 x 3.5 centimeter (cm) column and equilibrated overnight with 100 mM monosodium phosphate ($NaHPO_4$) pH 7.0 One-third (∿ 7 ml) of the ammonium sulfate precipitated, dialyzed material was applied to the Protein A-agarose column and the column was washed with 100 mM phosphate buffer, until the optical density of the eluate taken at 280 nm ($OD_{280}$) was at background levels. The

immunoglobulin G fraction of the antitheophylline antisera was eluted with 1 M acetic acid. Those fractions which had a high optical density at 280 nm ($OD_{280}$) were pooled and dialyzed against a solution containing 0.14 M sodium chloride and 0.05 M citrate at pH 5.8. The concentration of the purified antibody was 5.62 mg of protein/ml of solution.

D. Purity and Activity of Anti-Theophylline Antibody

Addition of anti-theophylline antiserum to the zymogen activation system led to positive interferences, due to the presence of activated blood coagulation factors in the serum sample. Protein A-purified anti-theophylline antibodies contributed neither positive nor negative interferences to the system. The purified antibody retained its ability to interact with theophylline, and showed a single precipitin line on radial immunodiffusion when tested against Factor X-theophylline conjugates.

Method and Results

A. Enzyme Assay

All assays were performed in a total volume of 1 mL which contained 15 mM tris(hydroxymethyl)-aminomethane hydrochloride buffer at pH 8.2, 70 mM sodium chloride and 7.5 mM calcium gluconate. The Factor X activating enzyme from Russell's Viper Venom (RVV, purchased from Sigma Chemical Co., St. Louis, MO) catalyzed conversion of Factor X to Factor Xa. This reaction was assayed in solutions which contained 0.6 mM of a chromogen substrate S-2222 for Factor Xa, Factor Xa substrate, 0.2 NIH units of Factor X or Factor X-analyte conjugate and 0.01 to 0.05 units of RVV. All assays were run at 25°C and monitored continuously at 406 nm on an Hewlett Packard 8450

spectrophotometer. Absorbance was plotted versus $(\text{time in minutes})^2$ to yield a linear plot. One NIH unit of a blood coagulation factor is the amount which is equivalent to the amount of 1 ml of normal DEF human plasma. One unit of RVV is the amount of activating enzyme which will produce a clotting time of between 14 and 22 seconds in a one stage determination of Factor X, as described by Bachmann, F., *et al.*, *Thromb. et Diath. Haemorrh.* *2*, 24 (1958).

B. Kinetics of Zymogen Activation

The immunoassay reaction scheme is shown in Figure 1. When the zymogen, Factor X, was incubated with the chromogenic substrate, S-2222, for Factor Xa, very little color was formed. However, when RVV was added to catalyze the conversion of Factor X to Factor Xa, color development was rapid and nonlinear with respect to time. A series of equations were derived which predicted that (1) color formation in the system should be linear with the square of time and (2) the slope of the line generated by a plot of absorbance versus $(\text{time})^2$ should be a measure of the rate of the RVV catalyzed reaction. The rate of this reaction is, in turn, dependent upon the concentration of Factor X and/or RVV in the system. These predictions were verified experimentally using purified RVV and Factor X.

When anti-theophylline antibody was added to the zymogen system, activity [measured as slope of absorbance versus $(\text{time})^2$] decreased as antibody concentration increased, as shown in Figure 2. At high concentrations of antibody (200-400 μg/mL), activity in the system was decreased to less than 10 percent of control values.

An antibody concentration (121 µg/mL) was chosen which inhibited the system by approximately 75 percent and a dose response curve was generated by the addition of free theophylline to the system. As shown in Figure 3, when free theophylline at concentrations between 5 and 50 micromolar was included in the assay, inhibition by antibody was relieved, and the activity of the system [measured as the slope of a plot of absorbance versus $(\text{time})^2$] was restored. It was therefore possible to generate a dose response curve for the analyte, theophylline, using the zymogen activator system.

*Example 2*

A specific binding assay for the determination of theophylline as the analyte (A) can be performed using chymotrypsinogen/trypsin as the substrate/first enzyme pair. The enzymatic reaction used is basically

$$\text{Chymotrypsinogen} \xrightarrow{\text{trypsin}} \text{chymotrypsin}$$

substrate → detectable response

wherein the chymotrypsinogen is the first substrate, trypsin is the first enzyme and chymotrypsin is the second enzyme or enzyme product.

Chymotrypsin hydrolyses the substrate, N-benzyloly-L-tyrosine ethyl ester, to produce a detectable response (a higher absorbance at 256 mn). The assay utilizes a modulator of the enzymatic reaction, soybean trypsin inhibitor, as the label which inhibits the production of chymotrypsin thereby decreasing the detectable response. Soybean trypsin inhibitor is used as the label to form the detectant reagent, A-I.

If no analyte is present, the antibody to the analyte, Ab, will bind the detectant reagent, A-I (herein referred to as the inhibitor), and decrease the inhibitory effect of soybean trypsin inhibitor, thereby increasing the detectable response of the system. If analyte is present, the antibody to the analyte, Ab, will bind the analyte, leaving the detectant reagent, A-I, to exert its full inhibitory effect on the enzymatic response of the system.

Materials

A. Preparation of a soybean trypsin inhibitor: 8-(3-carboxypropyl)-theophylline conjugate

The detectant reagent is prepared by linking soybean trypsin inhibitor to an analogue of theophylline, 8-(3-carboxypropyl)-theophylline. The lactam of 8-(3-carboxypropyl)-theophylline is prepared by the method of Cook *et al.*, *Res. Comm. Chem. Pathol. Pharmacol.* *13*, 497 (1976). Ten milligrams (0.45 micromole) of soybean trypsin inhibitor (available from Sigma Chemical Co., St. Louis MO) in 3 mL of 2.0 M sodium bicarbonate is combined with 10.7 mg (0.45 millimole) of the lactam in 100 µL of dimethylformamide. This reaction mixture is allowed to stand at room temperature for 1 hour and then at 4°C for 5 hours. After standing, the reaction mixture is chromatographed on a 2.5 x 28 cm column of Sephadex® G-25 (medium) (Pharmacia Fine Chemicals, Piscataway, N.J.) and equilibrated with 80 mM tris(hydroxymethyl) aminomethane (tris-hydrochloride) buffer, pH 7.8. The chromatogram is developed with this buffer and 1 mL fractions are collected. The fractions containing the first peak of protein, detected by absorbance at 280 nm, are pooled and stored at 4°C.

B. Preparation of antibody to theophylline

Antibody to theophylline is prepared as described by Li *et al., Clin. Chem.* 27 (1981). The immunoglobulin G, IgG, fraction is isolated by affinity chromatography on Protein A Sepharose (Pharmacia Fine Chemicals). A 1 mL sample of the antiserum is applied to the 1 x 6 cm column of the affinity resin and the column is washed with 50 mM N,N-bis[2-hydroxyethyl]glycine (Bicine) buffer, pH 8.3, containing 0.2 M NaCl until the absorbance of the effluent at 280 nm is less than 0.05. The bound IgG is then eluted with 0.1 M glycine buffer, pH 3.0, and 1 mL fractions are collected in tubes containing 0.1 mL of 1 M Bicine buffer, pH 8.3. Fractions with absorbances greater than 0.2 at 280 nm are pooled and dialyzed against 80 mM tris-hydrochloride buffer, pH 8.0, containing 0.1 M calcium chloride.

C. Preparation of other reagents

Chymotrypsinogen A (from bovine pancreas, Sigma Chemical Co.) is dissolved at concentration from 2 to 50 μg/ml in 80 mM tris-hydrochloride buffer, pH 7.8, containing 0.1 calcium chloride and stored at 4°C.

Method and Results

A. Assay for chymotrypsin activity

Chymotrypsin hydrolyzes the ester groups of N-benzolyl-L-tyrosine ethyl ester, the product having a higher absorbance at 256 nm than the ester. The ester is not hydrolyzed by trypsin, thus the rate of increase in absorbance at 256 nm is a specific measure of chymotrypsin activity. The assay is conducted at 25°C in 80 mM tris-hydrochloride buffer, pH 7.8, containing 0.1 M calcium chloride.

B. Titration of trypsin

Chymotrypsinogen A has no enzyme activity but trypsin hydrolyzes a peptide bond to form reactive Π-chymotrypsin as outlined below:

Π chymotrypsin
trypsin
Chymotrypsinogen A
Π chymotrypsin
trypsin
neo-chymotrypsinogen → α-chymotrypsin
or
α-chymotrypsin

The new formed Π-chymotrypsin can act on chymotrypsinogen A to form neo-chymotrypsinogen which is converted to the active α-chymotrypsin by either trypsin or α-chymotrypsin.

The immunoassay readout system is first optimized by combining various concentrations of the trypsin with a fixed amount of chymotrypsinogen A, and then measuring chymotrypsin activity. This optimization is conducted with molar ratios of chymotrypsinogen A to trypsin ranging from 50 to 50,000.

The trypsin and chymotrypsinogen are combined and incubated at 25°C for 5 minutes. The ester substrate is added and the reaction rate is recorded. The chymotrypsinogen A/trypsin molar ratio which gives a substantial reaction rate, measurable over a 1 to 3 minute period, is used for further experimentation.

C. Titration of soybean trypsin inhibitor-theophylline conjugate

A range of concentrations of soybean trypsin inhibitor-theophylline conjugate are combined, in the 80 mM tris-hydrochloride buffer, 0.1 M calcium chloride, at the optimal concentration of trypsin determined as in part B above. The molar ratio of trypsin to inhibitor used is 0.2 to 5.0. Five minutes after combining the trypsin and inhibitor, chymotrypsinogen A is added. After 5 minutes further incubation, the substrate is added and the reaction rate is recorded. A concentration of inhibitor-theophylline conjugate which produces 80% inhibition of the activation of chymotrypsinogen A is used for further experiments.

D. Titration of antibody to theophylline

Various concentrations of antibody to theophylline are combined with the optimal level of inhibitor-theophylline conjugate in 80 mM tris-hydrochloride buffer, 0.1 M calcium chloride. Trypsin and chymotrypsinogen A are then added as outlined above (part C). Chymotrypsin activity is measured. The activity increases as increasing levels of antibody inactivate larger portions of the inhibitor-theophylline conjugate. The antibody concentration which recovers 80% of the chymotrypsin activity, measured in the absence of inhibitor, is used for further measurements.

E. Competitive binding assay for theophylline

Various concentrations of theophylline are combined with the optimal level of antibody as determined in part D. These reactions are set up in the 80 mM tris-hydrochloride buffer, 0.1 M calcium chloride. Inhibitor-theophylline conjugate, trypsin and chymotrypsinogen A are then added as described in part A.

Finally, substrate is added and the reaction rate is recorded. As the theophylline level is increased, the chymotrypsin activity will decrease.

*Example 3*

A specific binding assay for the determination of biotin was devised using Factor X/Factor X activating enzyme from Russell's Viper Venom as the first substrate/enzyme pair and prothrobin as the second substrate. Factor X is a zymogen which is cleaved by a protease present in Russell's Viper Venom (referred to herein as Factor X activating enzyme from Russell's Viper Venom and abbreviated RVV) to form the enzyme, Factor XA. Factor Xa subsequently cleaves prothrombin, a second zymogen present in the reaction mixture to form the active enzyme, thrombin. The final enzyme product, thrombin, is used in a detection system where it acts on a chromogenic substrate to produce a detectable response.

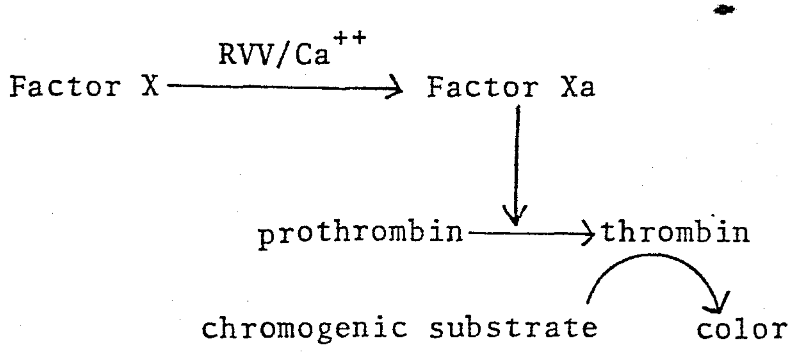

The first substrate, Factor X, was chosen as the label. The analyte, biotin, was conjugated with the carbohydrate side chain attached at the asparagine-36 of the heavy chain of bovine Factor X, forming the detectant reagent. In the presence of avidin, a protein which binds biotin, the reactivity of Factor X in the detectant reagent was inhibited; this inhibition could be competitively reversed by the addition of free biotin to the system (see Figure 6). The response of the double zymogen activation immunoassay system to changes in unbound biotin concentrations can be represented by the dose response curve for biotin. Activity of the system was measured by the addition of a chromogenic substrate for thrombin. The substrate, S-2238 (a product of Kabi Vitrum, AB and purchased in the U.S. from Helena Laboratories, Inc., Beaumont, TX), undergoes a color change when acted upon by thrombin. Absorbance changes with time were measured on a Hewlett-Packard Model 8450 spectrophotometer and absorbance was plotted against $(\text{time in minutes})^3$ to produce a linear plot. Details of experimental protocols and results follow.

Preparation of a Factor X-biotin Conjugate

A. Selective oxidation of sialic acid residues of Factor X

Two mg of bovine Factor X (purchased from Sigma Chemical Co., St. Louis, MO.) were dialyzed overnight with a buffer containing 0.1 molar (M) sodium acetate, pH 5.6 and 0.15 M sodium chloride. The resulting dialyzed solution was adjusted to contain 5 millimolar (mM) sodium metaperiodate and allowed to react for 10 minutes at 4 degrees centigrade (°C), 72 microliters (μL) of ethylene glycol were subsequently added. The reaction mixture was dialyzed overnight

against 2 liters of 10 mM sodium phosphate, pH 7.0, 0.15 M sodium chloride before further reaction.

B. Conjugation of oxidized Factor X to piperazine-biotin

To the dialyzed, oxidized material representing 0.254 micromoles (μmoles) oxidized sialic acid residues, was added 0.56 μmoles (determined as amino groups), of piperazine-biotin. (preparation follows)

The reaction mixture was allowed to incubate at 25°C for 2 hours to form a Schiff's base. Then 1 milligram (mg) sodium cyanoborohydride in 100 μL PBS (a solution of 0.01 M sodium phosphate, pH 7.0, and 0.15 M sodium chloride) was added, and the Schiff's base was allowed to reduce overnight at 4°C.

C. Preparation of Biotin-piperazine

A solution containing 1.22 grams (gm) of biotin (5 millimoles), 863 mg of N-hydroxysuccinimide (7.5 millimoles), and 15 ml of dimethylformamide (DMF) were evaporated at 30°C, at a pressure of 0.1 millimeters (mm) of mercury. This process was repeated. The residue of the two evaporations was suspended in 10 milliliters (mL) of DMF, cooled to 0°C, and treated with a solution containing 1.135 gm of N,N'-dicyclohexylcarbodiimide (5.5 millimoles), in 7.5 mL of DMF. The resulting suspension was then stirred for 0.5 hour at 0°C, and allowed to warm to ambient temperature over 3 hours. The solution containing activated ester was filtered and added to a solution stirred at 0°C, containing 1.502 gm of 1-(3-aminopropyl)-4-(3-t-butoxycarbonylaminopropyl)-piperazine and 700 μl of triethylamine (511 mg or 5.05 millimoles) in 5 ml of DMF. The resulting reaction mixture was allowed to warm to ambient temperature with stirring overnight.

The reaction mixture was concentrated on a rotary evaporator at 30°C, 0.1 mm of mercury, to give 5.1 gm of a reddish oil. The oil was dissolved in methanol, absorbed onto 5 gm of a silica gel, $SiO_2$-60 (EM Reagents, Darmstadt, Germany), and evaporated at 40°C, 12 mm of mercury. The resulting powder was placed atop a chromatography column packed with 300 gm of $SiO_2$-60 which had been packed and equilibrated with a solvent mixture containing 90:10:1 parts by volume (v/v/v) of chloroform ($CHCl_3$), methanol and 28% ammonium hydroxide. The column was then eluted with 3 liters of the above solvent mixture, followed by elution with 2 liters of a solution containing 80:10:1 parts by volume (v/v/v) mixture of the same solvents. The column was eluted with a flow rate of 2 mL/minute. Fractions of 12 mL were collected. Fractions, 330-440 contained the product, which were then pooled and concentrated to give 1.14 gm of a light yellow glass (compound I).

Compound I:

CMR(22.5$MH_z$, $D_2O$)$\delta$:174.2, 162.8, 155.7 ppm

IR(KCl):1695, 1640$cm^{-1}$

MS(FAB,$X_e$):M/e 527(M + 1, 42.4%)

A solution containing 881 mg of Compound I (1.67 millimoles), in 20 ml of trifluoroacetic acid was stirred for 90 minutes at 0°C. The solvent was evaporated at 0°C, 0.1 mm of mercury, to give 1.15 gm of an oily residue. This residue was dissolved in 10 mL of water and adjusted to pH 8 with 28% ammonium hydroxide. The sample was then chromatographed on a 3.7 x 37 cm column of Waters® $C_{18}$ reverse phrase resin (40 micron mesh, obtained from Waters Associates, Inc., Milford, MA) eluted with water at a flow rate of 6 ml/min. Fractions of 12 ml were taken. Fractions

containing the product were combined with two pools on the basis of purity and concentrated to give 44 mg and 610 mg, respectively. The glassy solids were then purified by separate passes on a 2.5 x 60 cm column of Sephadex® LH-20 (obtained from Pharmacia Fine Chemicals, Uppsala, Sweden), eluted with water at a flow rate of 1 mL/min. Fractions containing the pure product were pooled and concentrated to give a total of 154 mg of the final product as a glass. The product referred to herein as Biotin-piperazine, was dissolved in $H_2O$ and was assayed for free amino groups by the method of Habeeb, A.F.S.A. *Anal. Biochem. 14*, 28 (1966), and for biotin by the method of Green, *M., Meth. Enzymol. 18A*, 418 (1970).

Biotin-Piperazine

CMR(22.5 $MH_z$, $D_2O$)$\delta$: 174.98, 160.08 ppm.
MS(FAB, Xe):m/e = 427 (M + 1, 62%).

D. Preparation of an avidin affinity column

Sepharose® CL-4B (purchased from Pharmacia, Inc. Piscataway, NJ) (10 g) was activated by the method of Wilchek, T., and Miron, T. *Biochem. Int. 629*, (1982); activation time did not exceed 15 minutes. 30 mg avidin (purchased from Sigma Chemical Co., St. Louis MO.) in 15 mL of 0.2 molar sodium bicarbonate, pH 8.4 was added to the activated resin. The material was rotated 40 hours at 4°C. The avidin-Sepharose was filtered and washed sequentially with 0.1 M sodium bicarbonate, pH 8.4, and water. 10 mL of 1.0 molar ethanolamine, pH 9.0 was added to the washed resin. The resin was rotated end to end for one hour, washed sequentially with 0.1 M sodium bicarbonate and PBS, and packed into a 1.5 x 15 cm chromatography/column.

The binding affinity and capacity of this avidin-Sepharose column was subsequently modified by treatment of the resin with guanidinium-hydrochloride as follows: two column volumes of 6 M guanidinium-hydrochloride were washed through the avidin column. The column was stoppered and allowed to incubate for approximately 18 hours at 25°C. The column was then washed with 6 M guanidinium-hydrochloride until the optical density at 280 nanometers ($OD_{280}$) of the eluate was below 0.01 (units). After guanidinium-hydrochloride treatment the column was washed sequentially with 2 column volumes of PBS, 2.0 millimolar biotin (purchased from Sigma Chemical Co., St. Louis MO) in PBS, 0.1 molar glycine, pH 2.0, and PBS, then stored at 4°C.

E. Purification of Factor X-biotin conjugate

After reductive amination, the sample was placed on a 1.25 x 25 cm chromatography column packed with Sephadex® G-25 (purchased from Pharmacia, Inc., Piscataway, N.J.), equilibrated with Tris-NaCl [15 mM tris-(hydroxymethyl)-aminomethane (Tris-HCl), pH 8.2, containing 70 mM sodium chloride] and eluted with the same buffer at 4°C to remove the excess biotin-piperazine. Fractions were assayed for protein content by determining the $OD_{280}$. Protein containing fractions were assayed for Factor X activity as described below. The active fractions were pooled for subsequent application to the avidin column.

Fractions containing Factor X activity were then applied to a 1 x 7 cm column of immobilized avidin. The column was eluted at 25°C with Tris-NaCl until no more protein, as determined by optical density measurements taken at 280 nm, was washed from the column. Factor X-biotin conjugate was specifically

eluted from the affinity resin with 2 mM biotin in Tris-NaCl. Fractions were assayed for Factor X activity and those fractions of high and intermediate activity were pooled separately. Total yield of Factor X activity was 47.7%. The pooled material was made 1 mg/mL with bovine serum albumin (obtained from Miles Scientific, Naperville, IL) and dialyzed overnight against Tris-NaCl to remove free biotin.

F. Stability of Factor X-Biotin Conjugate

The Factor X-biotin conjugate, prepared as described above, was tested over 5 days for its ability to retain Factor X activity. The pooled fractions with intermediate Factor X activity lost approximately 30% of their activity after the first day of storage, and subsequently stabilized. The pooled fractions with high Factor X activity lost less than 20% of their activity during the course of the stability studies and exhibited stability equivalent to that of untreated Factor X.

G. Determination of Equivalent Activity in Factor X-Biotin and Untreated Factor X

The specific activity of the Factor X-biotin conjugate was calculated based on its ability to be activated by RVV. Factor X-biotin conjugate with an activity equivalent to that of 0.25 NIH units per milliliter of untreated Factor X was used for subsequent experiments. (One NIH unit of a blood coagulation factor is defined as amount of blood coagulation factor equivalent to that found in one milliliter of normal human plasma). One unit of RVV is the amount of enzyme which will produce a clotting time of between 14 and 22 seconds in a one

stage determination of Factor X, as described by Bachmann, F., *et al.*, *Thromb. et Diath. Haemorrh.*, *2*, 24 (1958).

H. Activity of the Factor X-biotin Conjugate in the Multiple Zymogen Activation System

Both pooled fractions were tested for their ability to react in the complete cascade. A replot of absorbance versus the cube of time (in minutes) was linear, indicating that the Factor X conjugated derivative had retained its ability to react properly with thrombin. The slope of this linear plot is used as a measure of the Factor X activity in the cascade system [Blake, D.A., Blake, R.C., II, and Smith P.J. *Fed. Proc. 42*, 1863 (1983)].

Addition of the biotin-binding protein, avidin, to the system resulted in a decrease of color formation, with a retention of proper kinetic response. Titration of the Factor X-biotin cascade with avidin is shown in Figure 5. Inhibition of the system was linear when 2 to 10 micrograms/milliliter (μg/mL) of avidin were added; at very high concentrations of avidin (200-500 μg/mL) inhibition reached a maximum of approximately 85%.

An avidin concentration (8 μg/mL) was chosen which produced approximately 50% inhibition of cascade activity, and free biotin was added in an attempt to assess the sensitivity of the system to analyte concentration, as shown in Figure 6. These results indicate that a linear dose-response curve with the addition of 20 to 100 nanomolar biotin.

I. Enzyme Assays

RVV and prothrombin were purchased from Sigma Chemical Co., St. Louis, MO. All assays were performed in 1 mL total volume which contained 15 mM Tris-H 1, pH 8.2, 70 mM sodium chloride and 10 mM calcium chloride. RVV catalyzed conversion of Factor X to Factor Xa and was assayed in solutions which also contained 0.6 mM S-2222 (Factor Xa substrate, a product of Kabi Vitrum, AB and purchased from Helena Laboratories, Beaumont, TX), 0.05 to 0.10 units of RVV and varying concentrations of Factor X or Factor X-analyte conjugate. Multiple zymogen activation was assayed in solutions which contained buffer salts as described above, 20 μg/mL phospholipid, 0.075 mM S-2238 (a thrombin substrate), 0.2 NIH units of prothrombin, 0.05 units of RVV and Factor X-analyte conjugate with activity equivalent to that of 0.25 NIH units of untreated Factor X. Analyte binding protein was allowed to incubate 5 minutes at 25°C with Factor X-analyte with and without free analyte before initiation of the reaction. Phospholipid solutions were made fresh weekly by sonication of a 2 mg/mL solution of L-alpha-phosphotidylcholine (purchased from Sigma Chemical Co., St. Louis, MO.) in Branson sonicator (50 milliamperes, 2 x 2 minute bursts).

All assays were run at 25°C and monitored continuously at 406 nm on Hewlett Packard 8450 spectrophotometer. Depending upon the reaction monitored, absorbance was plotted vs. $(\text{time in minutes})^2$ for the activation of Factor X, or in $(\text{time in minutes})^3$ multiple for the zymogen activation, to yield linear plots.

Obviously, many other modifications and variations of the invention as hereinbefore set forth may be made without departing from the spirit and scope thereof.

Claims:

1. A specific binding assay method for determining an analyte in a test sample, which method comprises the steps of:

(a) combining the test sample with a reagent system including a detectant reagent which is incorporated with a label, resulting in the formation of a bound-form of the detectant reagent and an unbound-form of the reagent, and

(b) determining the detectant reagent in the bound-form or in the unbound-form by means of the label as a function of the analyte in the test sample,

characterized in that the label is a participant in an enzymatic reaction wherein a first enzyme acts on a first substrate to produce a second enzyme, the label being selected from the first substrate or a cofactor or modulator of the first enzyme.

2. The method of claim 1 characterized in that the second enzyme acts on a second substrate to produce a third enzyme.

3. The method of claim 1 or 2 characterized in that either the first substrate is a zymogen or the second enzyme is formed by the addition of a chemical group to the first substrate or by the cleavage of a chemical group from the first substrate, which addition or cleavage is catalyzed by the first enzyme.

4. The method of any of claims 1 to 3 of the homogeneous type wherein the detectant reagent is measured without separation of the bound- and unbound-forms thereof.

5. The method of claim 4, which method comprises the steps of:

(a) forming a reaction mixture by combining the liquid medium with a reagent system including (i) a binding partner for the analyte, and (ii) the detectant reagent which comprises the analyte, or a binding analog of the analyte, incorporated with a label, whereby there results in the reaction mixture a bound-form of the detectant reagent, wherein the detectant reagent is bound to the binding partner, and an unbound-form of the detectant reagent, wherein the detectant reagent is not so bound, the label having a detectable characteristic which is measurably different when the detectant reagent is in the bound-form compared to the unbound-form, and

(b) measuring the detectable characteristic of the label in the reaction mixture as a function of the analyte in the liquid medium.

6. The method of any of claims 1 to 3 of the heterogeneous type wherein the bound- and unbound-forms of the detectant are separated and the label measured in one thereof.

7. A reagent system for use in the specific binding assay determination of an analyte in a test sample, which system comprises a detectant reagent which is incorporated with a label, the combination of the reagent system and the test sample resulting in the formation of a bound-form of the detectant reagent and an unbound-form of the detectant reagent, determination of the detectant reagent in the bound-form or in the unbound-form by means of the label being a function of the analyte in the test sample,

characterized in that the label is a participant in an enzymatic reaction wherein a first enzyme acts on a first substrate to produce a second enzyme, the label being selected from the first substrate or a cofactor or a modulator of the first enzyme.

8. The reagent system of claim 7 characterized in that the second enzyme acts on a second substrate to produce a third enzyme.

9. The reagent system of claims 7 or 8 characterized in that either the substrate is a zymogen or the second enzyme is formed by the addition of a chemical group to the first substrate or by the cleavage of a chemical group from the first substrate, which addition or cleavage is catalyzed by the first enzyme.

10. The reagent system of any of claims 7 to 9 in the form of a test kit for use in the homogeneous immunoassay determination of an analyte in a liquid medium, comprising:

(a) a binding partner for the analyte, and
(b) a detectant reagent comprising the analyte, or a binding analog of the analyte, incorporated with the label.

Zymogen Activation Immunoassay System:

Zymogen Label

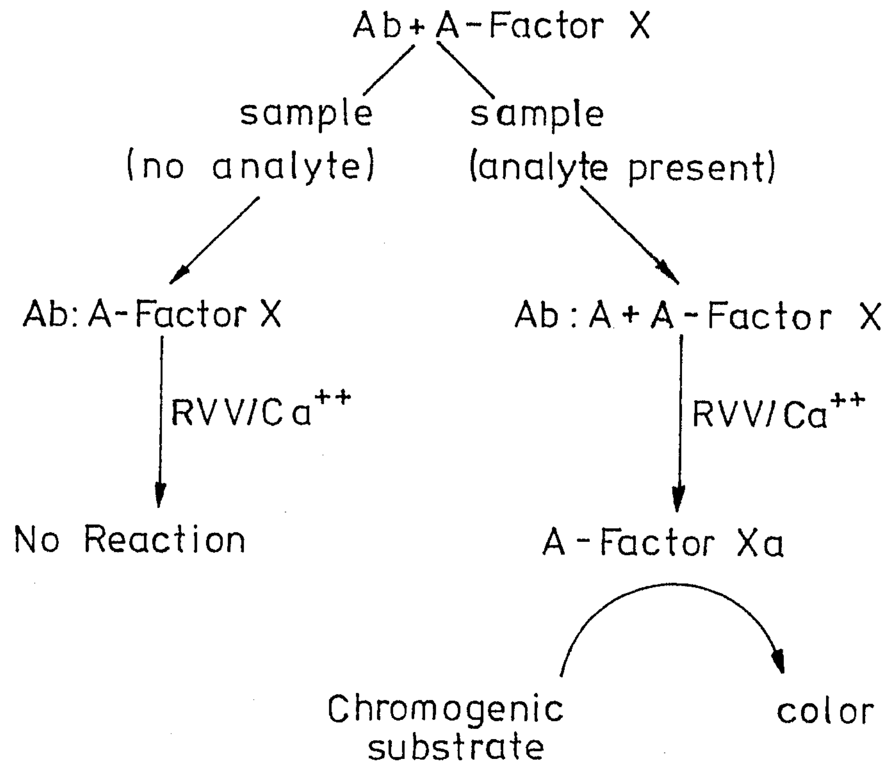


FIG. 1

ANTIBODY TITRATION OF ZYMOGEN ACTIVATION IMMUNOASSAY SYSTEM

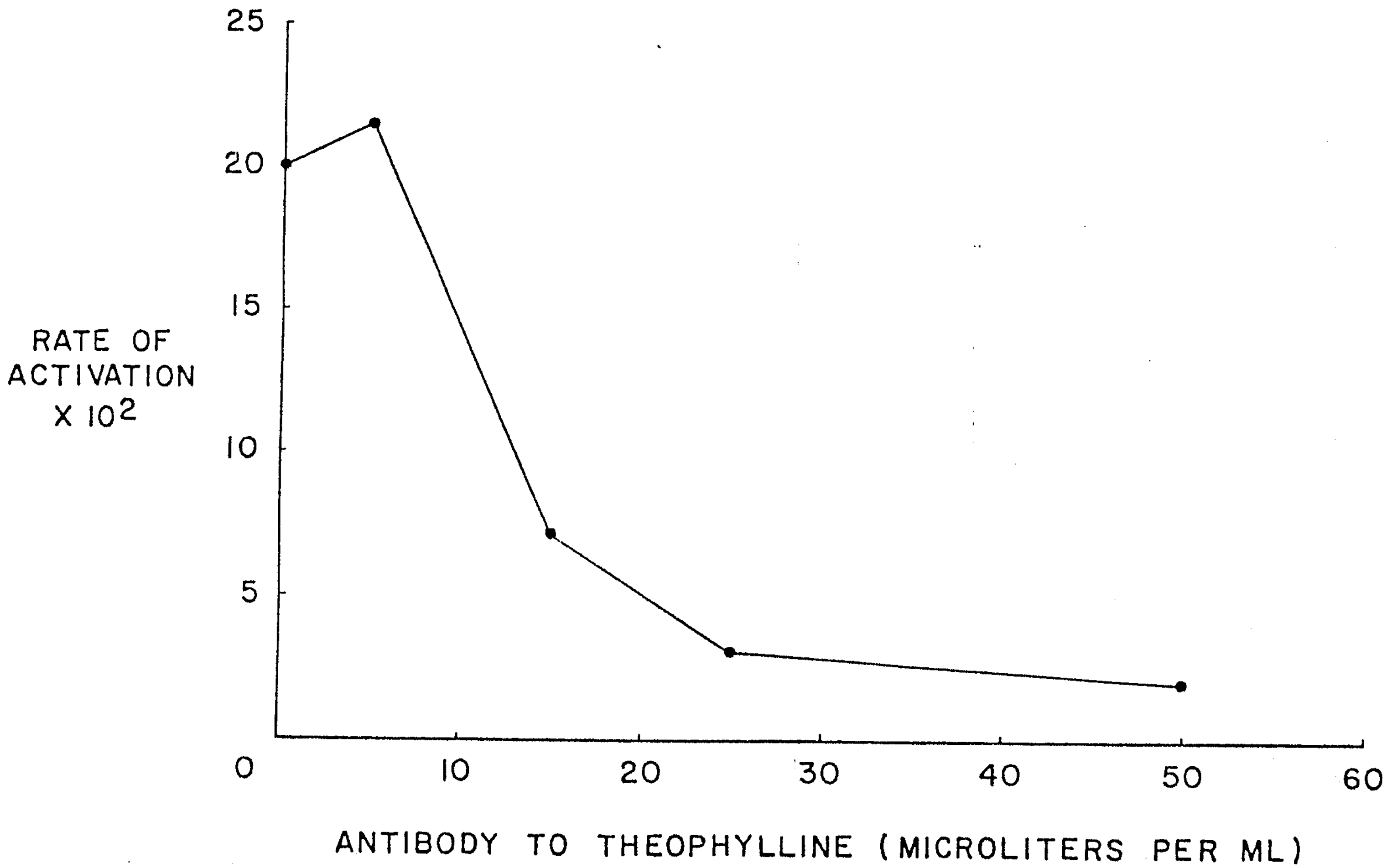


FIG. 2

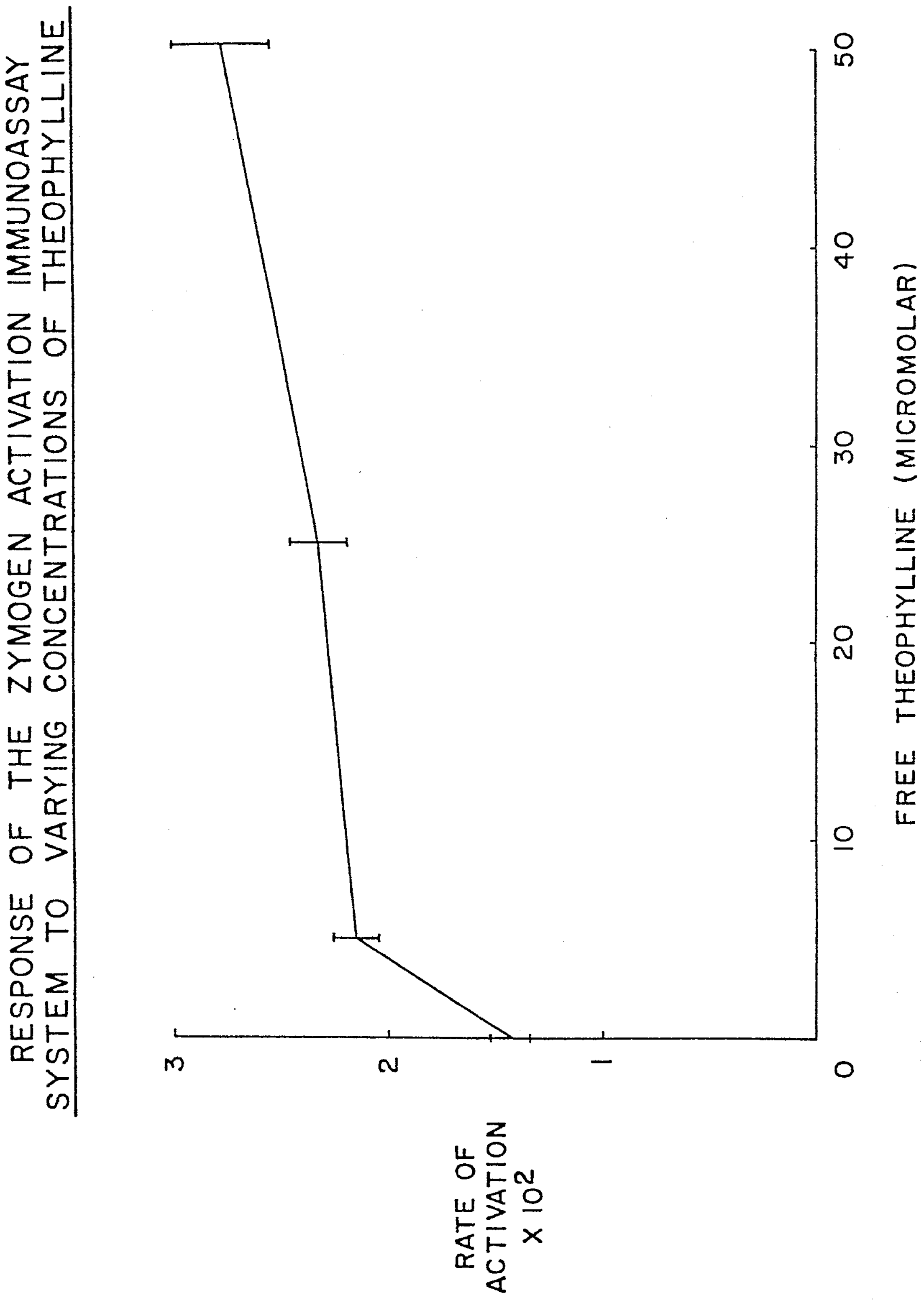
RESPONSE OF THE ZYMOGEN ACTIVATION IMMUNOASSAY
SYSTEM TO VARYING CONCENTRATIONS OF THEOPHYLLINE
RATE OF
ACTIVATION
X 10²
3
2
1
0
10
20
30
40
50
FREE THEOPHYLLINE (MICROMOLAR)

FIG. 3

4/6

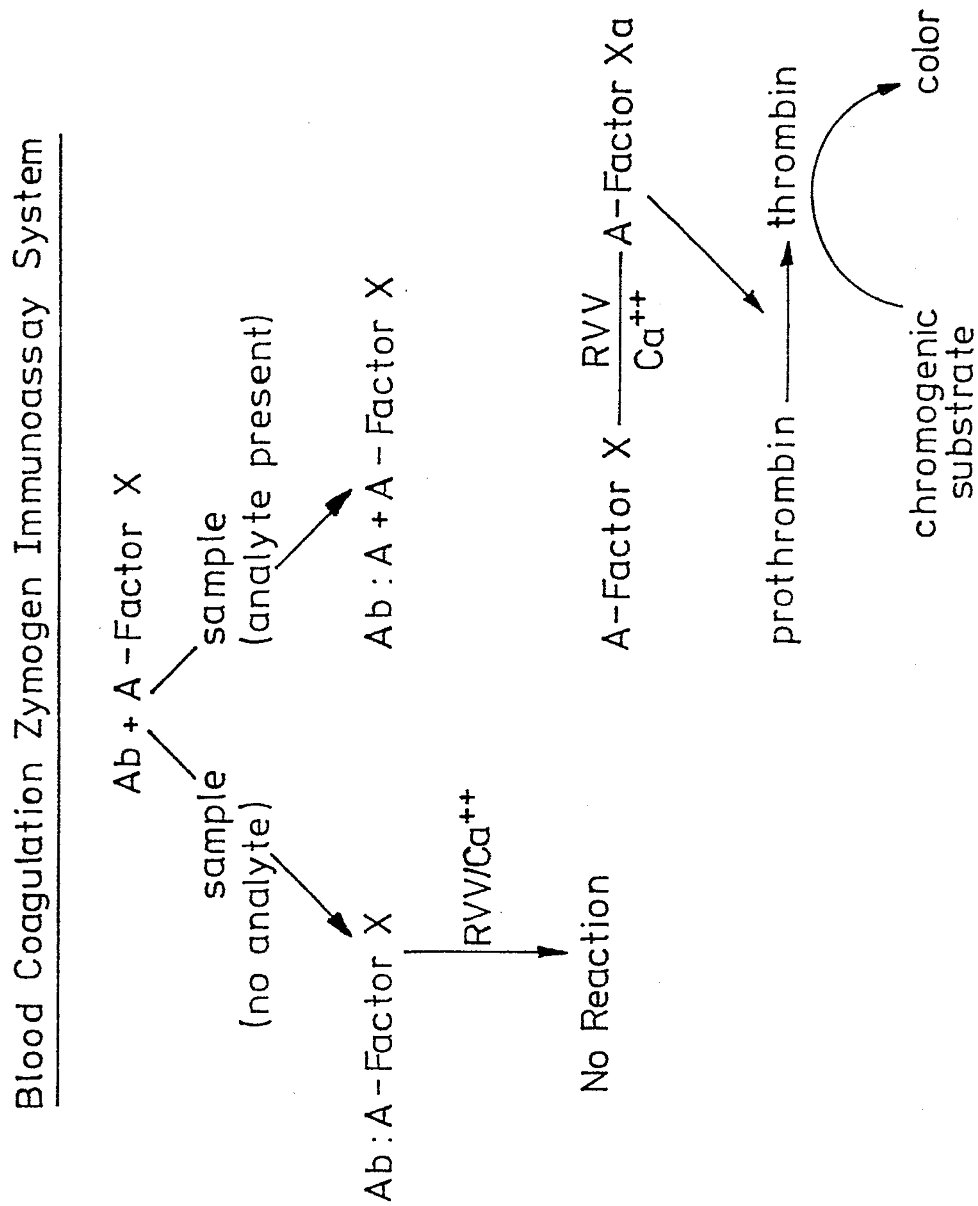

Blood Coagulation Zymogen Immunoassay System
Ab + A - Factor X
sample
(no analyte)
sample
(analyte present)
Ab : A - Factor X
RVV/Ca++
No Reaction
Ab : A + A - Factor X
A-Factor X
RVV
Ca++
A-Factor Xa
prothrombin
thrombin
chromogenic
substrate
color


FIG. 4

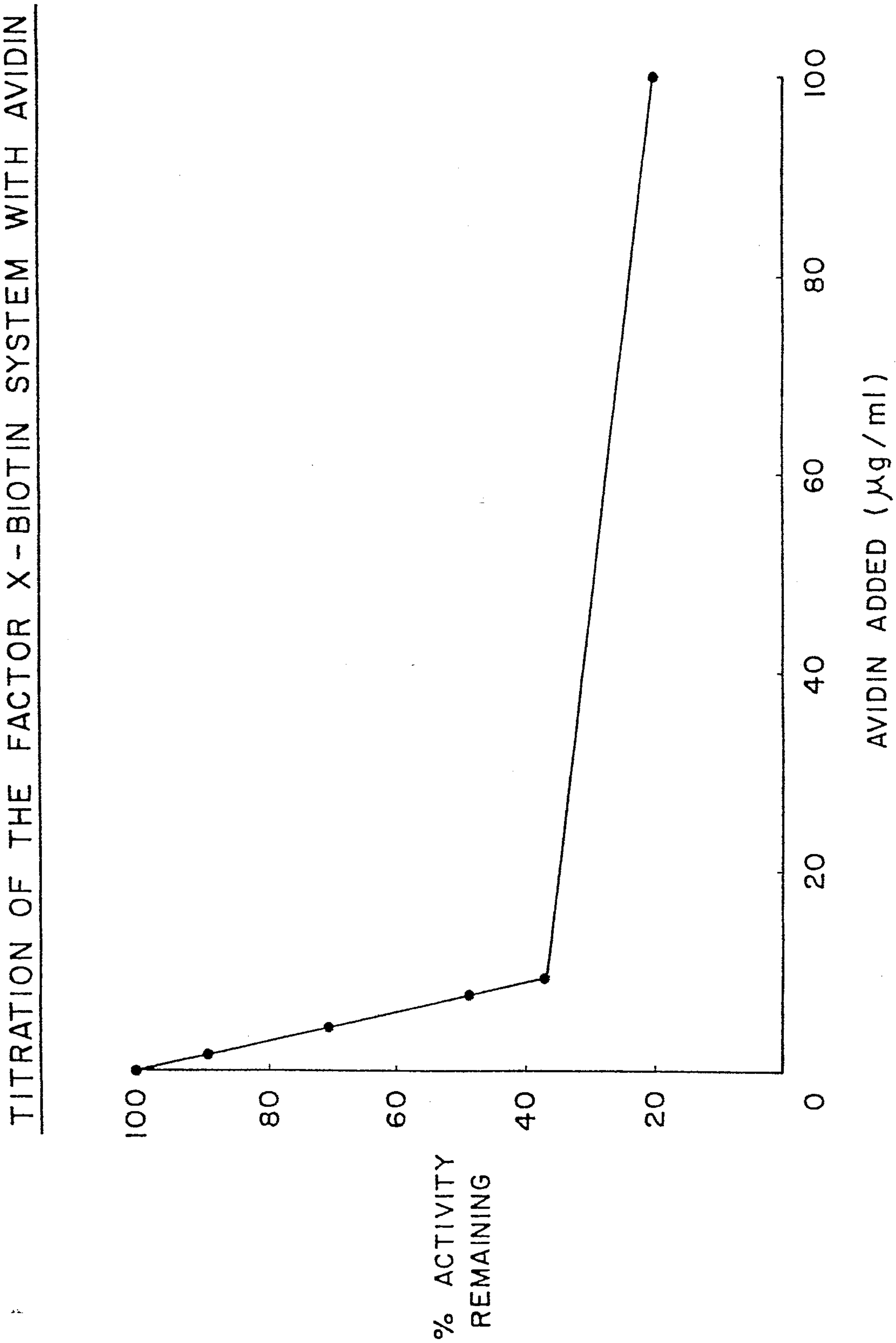
TITRATION OF THE FACTOR X-BIOTIN SYSTEM WITH AVIDIN
% ACTIVITY REMAINING
100
80
60
40
20
0
20
40
60
80
100
AVIDIN ADDED (μg/ml)

FIG. 5

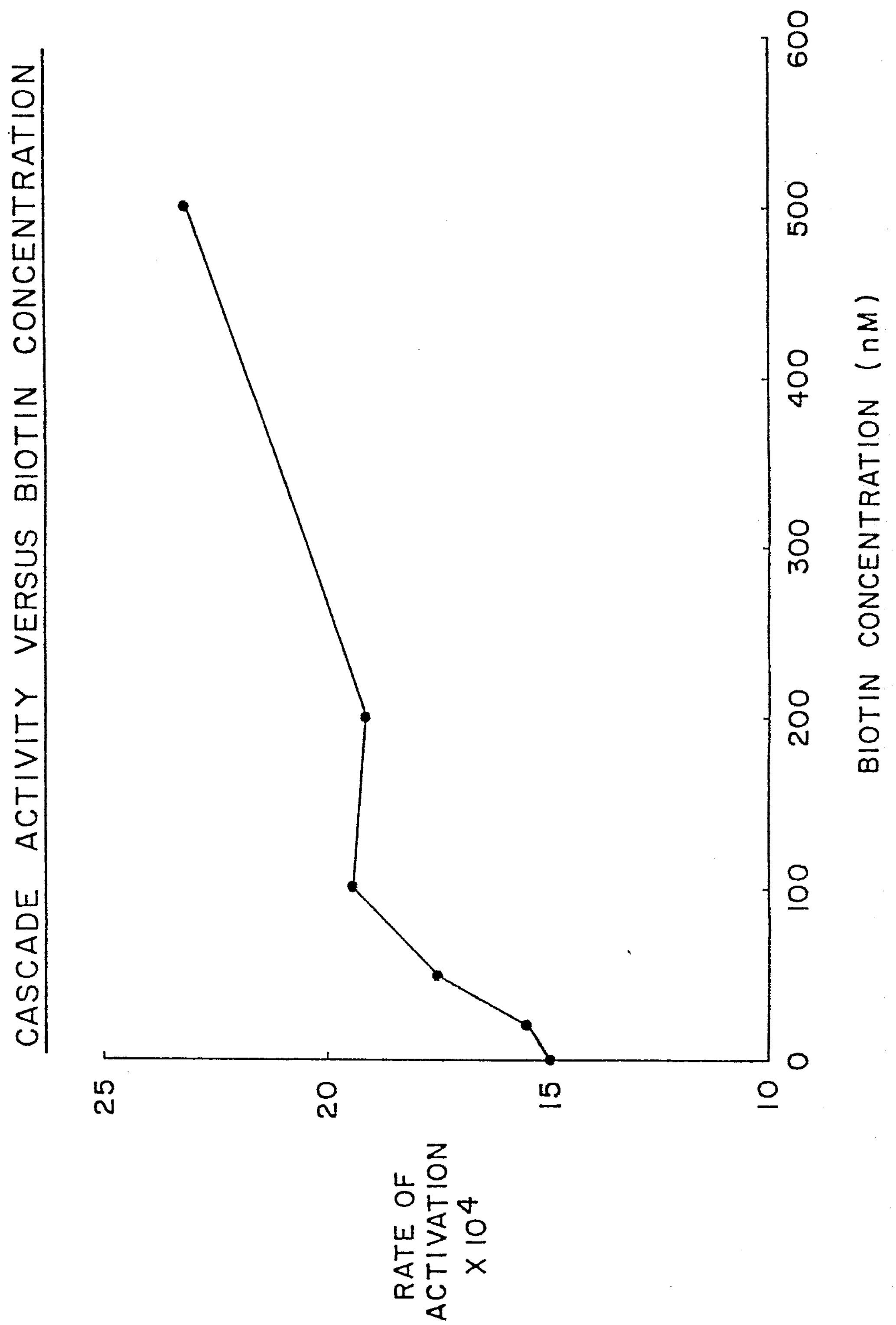
CASCADE ACTIVITY VERSUS BIOTIN CONCENTRATION
RATE OF ACTIVATION X 10^4
25
20
15
10
0
100
200
300
400
500
600
BIOTIN CONCENTRATION (nM)

FIG. 6